# EUROPÄISCHE PATENTSCHRIFT

(11) **EP 3 214 992 B1**
(45) Veröffentlichungstag und Bekanntmachung des Hinweises auf die Patenterteilung: **23.06.2021**
(21) Anmeldenummer: 15790924.3
(22) Anmeldetag: 04.11.2015
(51) Int. Cl.: A61B 1/307, A61M 25/09, A61B 5/03, A61B 5/00, A61B 5/01, A61B 5/0215, A61B 17/22, A61M 25/00, A61B 5/20

(54) **ÜBERWACHUNGSSYSTEM**
MONITORING SYSTEM
SYSTEME DE SURVEILLANCE

(30) Priorität: 06.11.2014 DE 102014116221
(43) Veröffentlichungstag der Anmeldung: 13.09.2017
(73) Patentinhaber: Ferton Holding S.A., 2800 Delémont (CH)
(72) Erfinder: TRAXER, Olivier, 75020 Paris (FR)
(74) Vertreter: Müller Schupfner & Partner Patent- und Rechtsanwaltspartnerschaft mbB
(86) Internationale Anmeldenummer: PCT/EP2015/075671
(87) Internationale Veröffentlichungsnummer: WO 2016/071383

(56) Entgegenhaltungen:
- WO-A1-96/07351
- WO-A1-2014/145469
- WO-A1-2014/145469
- JP-A- 2010 057 541
- US-A1- 2009 287 048
- US-A1- 2010 198 191
- US-A1- 2013 237 864
- US-A1- 2014 275 950
- US-A1- 2014 275 950
- US-A1- 2014 276 036
- US-A1- 2014 276 036
- US-A1- 2014 276 110

## Beschreibung

Die vorliegende Erfindung betrifft ein Überwachungssystem für endoskopische Behandlungen, insbesondere zur Anwendung bei endourologischen Eingriffen.

In der Endourologie wird, um die Linse des Endoskops wie auch die Behandlungszone zu reinigen und eine gute Sicht zu ermöglichen, ein Spülstrom verwendet, welcher über einen Arbeitskanal des Endoskops bzw. einen separaten Spülkanal bereitgestellt wird. Bei der Weiterentwicklung der verwendeten Instrumente bzw. Endoskope ist man bemüht, mit immer kleiner werdenden Durchmessern zu arbeiten, um die Traumatisierung des Gewebes so gering wie möglich zu halten. Damit werden auch die vorhandenen Arbeitskanäle der Instrumente, die z. B. zum Spülen aber auch zum Absaugen verwendet werden, immer kleiner. Hohe Spülströme, z. B. kurzzeitig und mit hohem Druck für ein Freispülen der Behandlungszone, bedingen daher schnell einen signifikanten Druckanstieg im Inneren des Körpers, da die Flüssigkeit nicht schnell genug durch die kleineren Arbeitskanäle abgesaugt werden kann. Da die Niere ein sehr drucksensibles Organ ist, können bereits geringe intrarenale Druckanstiege zu glomerulären Nierenschädigungen und in Folge zu Niereninsuffizienz führen. Bei einem zu hohen intraoperativen Druck besteht zudem die Gefahr, dass kontaminierte Flüssigkeit aus dem Harntrakt vom Körper vermehrt absorbiert wird und es zu postoperativen Traumas oder sogar zu einer Sepsis kommt. Andererseits ist auch ein gewisser Überdruck in den zu untersuchenden Hohlorganen notwendig, um diese für einen besseren instrumentalen und optischen Zugang zu dilatieren und ein Kollabieren während des Eingriffes zu verhindern.

Ferner offenbart die US 2009/0287048 A1 einen Führungsdraht mit einer Bilderfassungseinrichtung, wobei eine Pumpe dazu vorgesehen ist, den Bereich, der bildlich erfasst werden soll, zu spülen. Hierzu weist der Führungsdraht beispielsweise Drucksensoren auf. Auf Basis der durch die Drucksensoren erfassten Werte kann die Pumpe gesteuert werden.

Die WO 96 07351 A1 betrifft einen Führungsdraht mit einem Drucksensor. Weitere Beispiele für Führungsdrähte, insbesondere mit Drucksensoren, sind in US 2010 198 191, US 2013 237864 A1 und JP 2010057541 A beschrieben.

In der US 2014/0276110 A1 werden Daten einer Messeinrichtung empfangen und mit einem Vergleichswert verglichen.

Aus der US 2014/0275950 A1 ist die Verwendung eines Ausgabesignals in Kombination mit einem Drucksensor bekannt.

In der WO 2014/145469 A1 ist offenbart, dass in dem Fall, dass ein Druckabfall erkannt wird, ein entsprechendes Signal ausgegeben wird. Dabei bezieht sich die WO 2014 145 469 A1 im Übrigen ebenfalls auf einen Führungsdraht mit einem Drucksensor.

Die US 2014/0276036 A1 offenbart die Ausgabe eines Warnsignals, wenn ein Schwellwert erreicht ist.

Es ist daher Aufgabe der vorliegenden Erfindung, ein Überwachungssystem für endoskopische Behandlungen, insbesondere für endourologische Eingriffe, anzugeben, welches eine sichere und einfache Erfassung von Umgebungsbedingungen, insbesondere Drücken und/oder Druckänderungen ermöglichen und die Gefahren für den Patienten minimieren.

Diese Aufgabe wird durch ein Überwachungssystem nach Anspruch 1 gelöst. Weitere Vorteile und Merkmale der Erfindung ergeben sich aus den Unteransprüchen sowie der Beschreibung und der beigefügten Figur.

Das Überwachungssystem der in Rede stehenden Art kann für alle Arten von endoskopischen Eingriffen eingesetzt werden, bei denen ein Spülstrom zum Einsatz kommt. Dies sind insbesondere die Behandlung von Nieren-, Harnleiter und Blasensteinen, die Behebung von Nierenstauchungen, Eingriffe an Prostata, Blase oder Harnröhre und die optische Kontrollen des Harnwegtrakts. Der Zugang kann dabei über natürliche Körperöffnungen erfolgen oder aber auch durch chirurgisch geschaffenen Öffnungen. Besonders hervorzuheben ist der Einsatz der Erfindung bei der Lithotripsie, wobei es keine Rolle spielt, welche Arten von Lithotripter eingesetzt werden. Zweckmäßigerweise umfasst das Endoskop zumindest einen Spülkanal, welcher ausgelegt ist, eine Linse des Endoskops bzw. die Behandlungszone mit einem Medium wie bspw. einer sterilen Flüssigkeit zu spülen, um die Sicht auf die Behandlungszone und insbesondere auf die zu zertrümmernden Körpersteine zu ermöglichen bzw. zu verbessern. Zudem kann das Endoskop auch zumindest einen Absaugkanal aufweisen, um z. B. Flüssigkeit aber auch Partikel (z. B. von den Körpersteinen) aus der Behandlungszone abzusaugen.

Mit Vorteil dient nun das Überwachungssystem dazu, die Umgebungsbedingungen in der Behandlungszone zu kontrollieren bzw. zu überwachen, wobei das Überwachungssystem hierzu mit Vorteil den Führungsdraht umfasst, welcher an die Behandlungszone geführt werden kann. Führungsdrähte der in Rede stehenden Art sind ihrem Grundaufbau nach aus dem Stand der Technik bekannt. Bevorzugt kommt ein Führungsdraht aus Metall oder Kunststoff (oder aus einer Mischung bzw. Kombination der beiden Materialien) zum Einsatz, welcher an seinem distalen Ende aus weicherem, flexiblerem Material besteht, um eine bestmögliche Flexibilität zu gewährleisten und die Gefahr von Verletzungen (beim Einführen in den Körper) zu minimieren. Zweckmäßigerweise ist der Führungsdraht zumindest bereichsweise hydrophil ausgebildet und/oder auch mit einer PTFE-Beschichtung (Polytetrafluorethylen) versehen um die Gleiteigenschaften zu verbessern. Der Führungsdraht weist einen Durchmesser von etwa 0,15 bis 3 mm, bevorzugt von etwa 0,4 bis 2 mm und ganz besonders bevorzugt von etwa 0,8 bis 1,2 mm auf. Im Übrigen kann hinsichtlich der geometrischen wie konstruktiven Ausgestaltung des Führungsdrahts auf die aus dem Stand der Technik bekannten Ausführungsformen verwiesen werden. Ähnliches gilt in Bezug auf die zumindest eine Messeinrichtung.

Abhängig davon, welcher Wert bzw. welche Daten erfasst werden sollen, wird z. B. ein entsprechender Druck-, Temperatur- oder Kraftsensor etc. eingebaut, der die Umgebungsbedingungen erfassen kann. Die Temperaturmessung kann ebenfalls über den Drucksensor erfolgen, für eine genauere Messung ist es aber von Vorteil, wenn der Temperatursensor separat ausgebildet ist und in der Nähe von aber beanstandet zu dem Drucksensor angebracht wird. Entscheidend ist, dass das Überwachungssystem die Auswerteeinheit umfasst, welche Daten, also insbesondere Messdaten, von der zumindest einen Messeinrichtung erfassen kann. Die Datenübertragung kann dabei sowohl kabelgebunden als auch kabellos bspw. über einen entsprechenden Transmitter erfolgen. Dabei umfasst die Auswerteeinheit zweckmäßigerweise den Komparator, der die von der Messeinrichtung empfangenen bzw. erfassten Daten mit einem Vergleichswert vergleicht. An dieser Stelle sei darauf hingewiesen, dass hierbei in der Regel nicht unmittelbar die Daten, welche von der Messeinrichtung empfangen werden, verwendet werden. So geben Sensoren in der Regel ein Spannungs- oder Stromsignal aus, welches erst in einen entsprechenden korrespondierenden Wert eines Drucks, einer Temperatur oder einer Kraft umgerechnet bzw. umgewandelt wird. Der Vergleichswert kann also konkret eine Temperatur, ein Druck oder eine Kraft sein. Ebenfalls kann der Vergleichswert aber auch ein rein technischer Wert wie bspw. ein Spannungssignal oder ein Stromsignal sein. Wichtig ist, das die Auswerteeinheit bzw. der Komparator die Daten mit dem Vergleichswert vergleicht und so z. B. erkennen kann, wenn die von der Messeinrichtung empfangenen Daten den Vergleichswert, ggf., um einen vorbestimmten Korrekturwert korrigiert, überschreiten / unterschreiten, da dann erfasst werden kann, ob die Umgebungsbedingungen in einem ungünstigen Bereich liegen, also z. B. eine Temperatur zu niedrig/hoch oder ein Druck zu niedrig/hoch ist.

Die Auswerteeinheit kann im Führungsdraht bspw. in/an einem proximalen Ende des Führungsdrahts angeordnet sein, sie kann aber auch über ein Kabel und eine entsprechende Datenleitung mit dem eigentlichen Führungsdraht bspw. auch über eine Steckverbindung verbunden werden. Zweckmäßigerweise ist die Auswerteeinheit in ein geeignetes Bedienteil/Handteil integriert, das vorteilhafterweise an dem proximalen Ende des Führungsdrahts angeordnet ist. Bezüglich der Auswerteeinheit sei noch darauf hingewiesen, dass es sich hierbei zweckmäßigerweise um eine kleine Recheneinheit wie einen Prozessor handelt. Der Komparator kann einen Teil oder ein Modul dieses Rechners sein, er kann aber auch durch die Funktionalität des Rechners gebildet sein also keine abgetrennte Einheit darstellen sondern lediglich durch ein Programm, welches auf dem Rechner ausgeführt wird, gebildet sein.

In einer bevorzugten Ausführungsform umfasst die zumindest eine Messeinrichtung einen Druck oder eine Druckänderung. Mit Vorteil kommt also ein Drucksensor zur Anwendung, wobei hierbei bevorzugt piezoresistive, piezoelektrische, kapazitive oder induktive Drucksensoren zum Einsatz kommen. Eine entsprechende Strom/Spannungsversorgung, welche gegebenenfalls für die Messeinrichtung(en) notwendig ist, kann über den Führungsdraht und ggf. über die bzw. von der Auswerteeinheit bereitgestellt werden. Bei dem Vergleichswert handelt es sich also zweckmäßigerweise um einen Druckwert. Bevorzugt kann also ein Druckwert oder ein Druckniveau vorgegeben werden, mit welchem die von der Messeinrichtung erfassten Drücke verglichen werden. Hierbei kann der Vergleichswert ein Absolutdruck sein, welcher z. B. nicht überschritten/unterschritten werden darf. Erfindungsgemäß ist der Vergleichswert ein Relativdruck , welcher sich auf ein Start-Druckniveau bezieht. Damit kann vorteilhafterweise leicht festgestellt werden, wenn ausgehend von einem Referenzwert (vgl. Start-Druckniveau) eine Druckschwelle überstiegen/unterschritten wird. Der Referenzwert stellt dabei erfindungsgemäß den Druckwert dar, der in der entsprechenden Körperzone/Behandlungszone vor Beginn der Behandlung herrscht. Er beträgt etwa 10 cm Wassersäule (1 kPa) bei endourologischen Eingriffen. Während eines solchen Eingriffs steigt der renale Druck durch das Saug-Spül-System auf Werte zwischen etwa 40 und 50 cm Wassersäule (4 bis 5 kPa). Steigt der Druck über 150 cm Wassersäule (15 kPa) hinaus, sind Schäden der Niere zu befürchten. Der Vergleichswert, der zweckmäßigerweise nicht überschritten werden sollte, beträgt nach aktuellen Erfahrungen etwa 100 bis 120 cm Wassersäule (10 bis 12 kPa) und kann, nach einer Alternative der Erfindung, für jeden Patienten individuell, mittels eines Korrekturwerts angepasst werden. Die Messeinrichtung sollte in der Lage sein, Druckwerte bis etwa 300 cm Wassersäule (30 kPa) auswerten zu können.

Nach einer weiteren bevorzugten Ausführungsform der vorliegenden Erfindung erfasst die zumindest eine Messeinrichtung eine Temperatur oder eine Temperaturänderung. Mit Vorteil kommt also ein Temperatursensor zur Anwendung, wobei hierbei bevorzugt Temperatursensoren auf Halbleiterbasis (sog. Chipsensoren) zum Einsatz kommen. Die Temperatur kann auch mittels des zuvor beschriebenen Drucksensors detektiert werden. Die vorbeschriebenen Messungen, der Referenzwert, der Vergleichswert, der Relativwert etc. zum Druck gelten mutatis mutandis auch für die Temperatur. Auch hier ist ein Vergleich mit einem Referenzwert wichtig, d.h. die Veränderung der Temperatur während des Eingriffs, weniger der absolute Temperaturwert.

Der Temperatursensor ist besonders bei Laserbehandlungen wichtig, die die Temperatur in der Behandlungszone, z.B. bei der Steinzertrümmerung in der Niere oder der Blase, i.d.R. erhöhen. Das kann z.B. durch eine Erhöhung des Spülmitteldurchlaufs ausgeglichen werden, obgleich das wiederum den Druck in der Behandlungszone erhöhen könnte. Es empfiehlt sich daher, beides zu messen, d.h. Druck und Temperatur, da ggf. auch die Laserleistung herabgesetzt oder ausgesetzt werden muss. Eine Temperaturerhöhung tritt z.B. auch dann ein, wenn der Spülmitteldurchlauf unterbrochen wird (z.B. wegen einer Leitungsverstopfung). Die durch die Auswerteeinheit gewonnen Daten zu Druck und Temperatur (siehe im Detail weiter unten) können dann auch für eine Regelung der Spülflüssigkeit bzw. des Behandlungsgeräts, wie z.B. des Lasers, genutzt werden.

Erfindungsgemäß umfasst die Auswerteeinheit eine Eingabeeinheit, über die der Vergleichswert eingestellt bzw. mittels Korrekturwert angepasst werden kann, wobei die Auswerteeinheit erfindungsgemäß auch eine Vielzahl von Vergleichswerten bzw. Korrekturwerten umfasst. Zweckmäßigerweise können also ein oder mehrere Vergleichswerte bspw. von einem behandelnden Arzt vorgegeben und angepasst werden. So kann bspw. ein Vergleichswert ein Absolutwert sein, welcher auf keinen Fall überschritten werden darf, da sonst eine Schädigung von Organen wie bspw. des Harnleitersystems droht. Ein weiterer Vergleichswert oder alternativer Korrekturwert kann ein patientenspezifischer Wert sein. Damit kann individuell auf einen Patienten Rücksicht genommen werden. So wäre es bspw. möglich, dass ein Patient äußerst sensibel oder vorbelastet ist, der Druck bzw. die Temperatur in der Behandlungszone aber noch weit von kritischen Werten entfernt ist. Durch das Überwachungssystem kann der behandelnde Arzt diese Unterschiede leicht und schnell feststellen und dementsprechend reagieren. Um den Vergleichswert anzupassen und bzw. um verschiedene Vergleichswerte vorzugeben weist die Auswerteeinheit bzw. deren Eingabeeinheit zweckmäßigerweise ein Touchpad oder entsprechende Bedienknöpfe auf. Insbesondere ist auch ein Display oder dergleichen vorgesehen, um den oder die eingestellten Vergleichswerte bzw. Korrekturwerte anzusehen bzw. zu überprüfen.

Bevorzugt umfasst die Auswerteeinheit eine Ausgabeeinrichtung, die ein Ausgabesignal erzeugt, welches erkennen lässt, wenn der Vergleichswert überschritten/unterschritten wird. Das Ausgabesignal kann ein entsprechender Warnton oder ein optisches Signal sein, welches den behandelnden Arzt aufmerksam macht. Möglich ist auch eine haptische Rückmeldung über ein Vibrieren oder Rütteln des bereits genannten Handteils/Bedienteils. Die Messinformationen können auch an eine Zentraleinheit übermittelt werden, die die Auswertung und Information des Anwenders übernimmt. Zweckmäßigerweise kann vorgegeben werden, ob bei einem Überschreiten oder bei einem Unterschreiten des Vergleichswerts das Ausgabesignal erzeugt werden soll. Der behandelnde Arzt kann auf Basis des Ausgabesignals vorteilhafterweise schnell reagieren und ggf. den Spülstrom reduzieren, die Absaug-Leistung erhöhen oder allgemein die Behandlung kurz aussetzen. Sowohl die Eingabeeinheit als auch die Ausgabeeinheit können als von der Auswerteeinheit getrennte Module ausgeführt sein, welche zweckmäßigerweise ebenfalls in dem Bedienteil/Handteil anordnet sein können.

Bevorzugt erzeugt die Auswerteinheit unterschiedliche Ausgabesignale, abhängig davon, welcher Vergleichswert bzw. Korrekturwert überschritten/unterschritten wird bzw. ist. Hierbei kann es auch möglich sein, unterschiedliche Ausgabesignale vorzusehen, je nachdem welcher Vergleichswert überschritten/unterschritten wird, bspw. durch unterschiedliche Tonhöhen oder unterschiedliche Farbsignale. Sind mehrere Messeinrichtung an unterschiedlichen Positionen am Führungsdraht vorgesehen, können die unterschiedlichen Ausgabesignale auch mit den unterschiedlichen Positionen der Messeinrichtungen korrelieren, um dem behandelnden Arzt einen Hinweis zu geben, wo ggf. Probleme zu erwarten sind. Das Ausgabesignal bzw. dessen Art und Gestaltung kann auch davon abhängig gemacht werden, ob eine Vergleichswert über- oder unterschritten wird.

Zweckmäßigerweise erfasst die Auswerteeinheit auch, wie oft und/oder wie lange der Vergleichswert überschritten/unterschritten wird. Bevorzugt erfasst die Auswerteeinheit auch, wie lange ein Vergleichswert überschritten wird. So ist es gegebenenfalls nicht notwendig bei einem einmaligen Überschreiten eines Vergleichswerts sofort ein Ausgabesignal zu erzeugen. Stattdessen erfasst die Auswerteeinheit zweckmäßigerweise wie oft, wie lange und/oder um welche Höhe ein Vergleichswert überschritten wird bzw. ist und gibt davon abhängig das Ausgabesignal aus. Es versteht sich, dass diese Funktionalität entsprechend durch einen Benutzer bspw. über die Eingabeeinheit parametriert werden kann. Grundsätzlich sei an dieser Stelle angemerkt, dass der Vergleichswert ebenso ein Vergleichsbereich sein kann. Es kann also durchaus von Vorteil sein, nicht einen konkreten Wert vorzugeben, sondern einen Bereich mit einem oberen und einem unteren Grenzwert. Dies ist insbesondere hilfreich, um auf Druckschwankungen zu reagieren, die auf die Messeinrichtung(en) bzw. deren Messgenauigkeit zurückzuführen sind.

Zweckmäßigerweise erzeugt die Auswerteeinheit ein Steuersignal für ein anderes Gerät, insbesondere für ein Saug-Spül-System, wenn der Vergleichswert überschritten/unterschritten wird bzw. ist. Bevorzugt hat bspw. das Überschreiten des Vergleichswerts über das Steuersignal einen direkten Einfluss auf den Spülstrom des Endoskops. Mit anderen Worten, kann also durch die Überwachungseinrichtung unmittelbar auf ein anderes Gerät wie hier bspw. das Saug-Spül-System und dessen Spülfunktion Einfluss genommen werden, wenn das Überwachungssystem erkennt, dass Vergleichswerte, mit anderen Worten also zulässige Schwellwerte, überschritten werden. Hinsichtlich des Drucks und/oder der Temperatur in der Behandlungszone könnte dieser somit schnell durch eine Reduzierung des Spülstroms oder eine Erhöhung der Absaug-Leistung reduziert werden. Es versteht sich, dass das Steuersignal sowohl kabelgebunden als auch kabellos übertragen werden kann.

In einer bevorzugten Ausführungsform wird die zumindest eine Messeinrichtung durch die Auswerteinheit kalibriert, insbesondere indem ein erfasstes oder empfangenes Druckniveau auf Null gesetzt wird. Dies ermöglicht ein äußerst einfaches und sicheres System. Das Prinzip wurde bereits im Zusammenhang mit dem Relativdruck-Vergleichswert erläutert. Vor dem Beginn des chirurgischen Eingriffs oder der Untersuchung, wenn das Endoskop bereits an die Behandlungszone (intrakorporal) geführt ist, wird die Messeinrichtung zweckmäßigerweise kalibriert. Mit Vorteil weist das vorab genannte Bedienteil/Handteil hierzu einen entsprechenden Hebel, Knopf oder Schalter auf. Zweckmäßigerweise ist diese Funktion aber auch in ein ggf. vorhandenes Touchpad integriert. Der in diesem Augenblick erfasste Druck bzw. Temperatur oder Kraft wird nun zu Null gesetzt bzw. als Referenzwert genommen und es kann leicht ein gegebenenfalls um einen Korrekturwert angepassten Vergleichswert vorgegeben werden, der einem Abstand von diesem Referenzwert entspricht. Zunächst wird also der "normale" Druck/Temperatur/Kraft in der Behandlungszone erfasst. Mit Vorteil erkennt das Überwachungssystem, wenn von diesem "normalen" Druck/Temperatur/Kraft abgewichen wird und meldet dies über das oder die Ausgangssignale. Diese Konfiguration ist äußert einfach und sicher und es bedarf nicht der Verwendung von in der Regel teueren und aufwendigen Absolutdrucksensoren bzw. entsprechenden Sensoren für Temperatur oder Kraft.

Bevorzugt ist die zumindest eine Messeinrichtung, also z.B. der Drucksensor, in einem Abstand von etwa 2 bis 5 cm zu dem distalen Ende des Führungsdrahts angeordnet. Die letzten 2 bis 3 cm eines Führungsdrahtes sind üblicherweise hoch flexibel ausgebildet, um eine bessere Steuerbarkeit und eine geringere Traumatisierung des umgebenen Gewebes zu erreichen. Der Drucksensor befindet sich daher bevorzugt nahe am distalen Ende des Führungsdrahtes, aber nicht innerhalb des hoch flexiblen Bereiches um dessen Funktion nicht zu beeinträchtigen. Erstreckt sich der hoch flexible Bereich über eine größere Länge, so ist der Drucksensor entsprechend weiter von dem distalen Ende des Führungsdrahtes entfernt angeordnet. Fehlt ein hoch flexibler Bereich, beträgt ein maximaler Abstand der zumindest einen Messenrichtung, insbesondere eines Drucksensors, von einem distalen Ende des Führungsdrahts weniger als etwa 20 mm, bevorzugt weniger als etwa 10 mm. Damit können bestmöglich die Umgebungsbedingungen und insbesondere also die Drücke und Druckänderungen in den Bereichen vor dem Sensor erfasst werden. Es ist aber auch vorstellbar, dass der Sensor innerhalb oder am proximalen Ende des Führungsdrahtes angeordnet ist und mit kommunizierenden Röhren innerhalb des Führungsdrahtes zu einer oder mehreren Öffnung im distalen Bereich des Führungsdrahtes verbunden ist. Abhängig von der entsprechenden Messtechnik kann die zumindest eine Messeinrichtung an einer Oberfläche wie auch im Inneren des Führungsdrahts angeordnet sein. Wird die Messeinrichtung an einer Oberfläche des Führungsdrahts angeordnet, können mit Vorteil auch dynamische Effekte und der Staudruck erfasst werden. Es versteht sich, dass auch eine Vielzahl von Messeinrichtungen vorgesehen werden kann, welche sowohl innerhalb als auch außerhalb des Führungsdrahts angeordnet sein können. Diese Messeinrichtungen müssen auch nicht alle vom gleichen Typ sein. Bei Verwendung mehrerer Messeinrichtungen können auch Profile, wie z. B. Druckprofile, Temperaturprofile oder Kraftprofile entlang der Führungsdrahts erzeugt werden. Beispielsweise kann es dadurch möglich sein, eine Aussage darüber zu treffen, inwiefern der Druck in der Behandlungszone abgebaut werden kann. Ebenso kann angezeigt werden, ob um die Behandlungszone herum etwaige Probleme auftreten. Grundsätzlich sei angemerkt, dass es äußert vorteilhaft sein kann, am Führungsdraht zumindest einen Lage- oder Positionssensor vorzusehen, um die Position und Lage des Führungsdrahts relativ zum Körper bestimmen zu können und damit ggf. den Ort eines erhöhten Drucks.

In einer alternativen Ausführungsform ist zumindest eine Messeinrichtung ein relativer Drucksensor und zumindest eine weitere Messeinrichtung ein absoluter Drucksensor, wobei der relative Drucksensor bevorzugt an einem distalen Ende des Führungsdrahts angeordnet ist, während der absolute Drucksensor bevorzugt an einem proximalen Ende des Führungsdrahts angeordnet ist. Zweckmäßigerweise kann der absolute Drucksensor dazu verwendet werden, den relativen Drucksensor zu kalibrieren.

Neben dem Drucksensor kann die weitere Messeinrichtung ein Temperatursensor sein, wobei der Temperatursensor ebenfalls bevorzugt an einem distalen Ende des Führungsdrahts, d.h. in der Behandlungszone, angeordnet ist, in der Nähe jedoch beabstandet vom Drucksensor. Der Abstand beträgt z.B. 2-10 mm, bevorzugt 3-6mm.

Ein Führungsdraht mit zumindest einer Messeinrichtung, welcher die vorgenannten Vorteile und Merkmale des Überwachungssystems und des Verfahrens aufweist, ist nicht Bestandteil der Erfindung.

Weitere Vorteile und Merkmale ergeben sich aus der nachfolgenden Beschreibung einer bevorzugten Ausführungsform des erfindungsgemäßen Überwachungssysmit Bezug auf die beigefügte Figur.

Es zeigt:

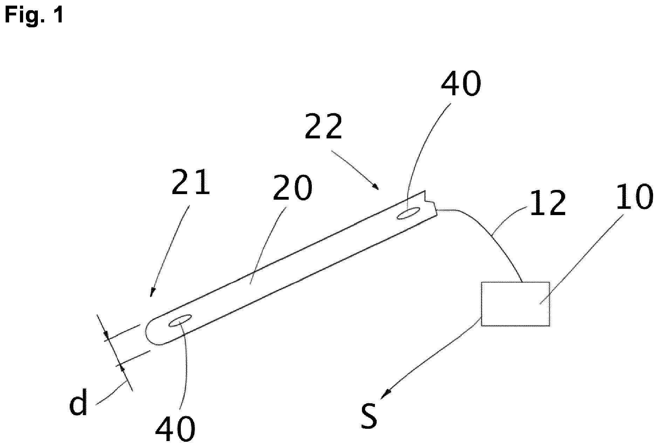

Fig. 1: eine schematische Darstellung einer bevorzugten Ausführungsform eines Überwachungssystems.

**Fig. 1** zeigt schematisch einen Führungsdraht 20, welcher an einem distalen Ende 21 eine Messeinrichtung 40 aufweist. An einem proximalen Ende 22 des Führungsdrahts 20 ist ebenfalls eine Messeinrichtung 40 vorgesehen. Über ein Kabel 12 ist der Führungsdraht 20 mit einer Auswerteeinheit 10 elektrisch verbunden. Alternativ bevorzugt kann die Verbindung auch kabellos erfolgen. Grundsätzlich muss die Auswerteeinheit 10 auch nicht derart getrennt von dem Führungsdraht 20 angeordnet sein, sondern kann als Teil dessen ausgebildet sein. Zweckmäßigerweise ist an einem Ende des Führungsdrahts 20 auch eine Bedienteil/Handteil vorgesehen, welches die Auswerteeinheit 10 umfassen. Ebenso kann in dem Bedienteil/Handteil auch eine Eingabeeinheit und eine Ausgabeeinheit vorgesehen sein, um sowohl Benutzereingaben zu ermöglichen als auch Ausgabesignale weiterzuleiten. Nicht dargestellt ist eine Strom- bzw. Spannungsversorgung, welche gegebenenfalls für die Messeinrichtungen 40 vorgesehen werden müssen. Ein Durchmesser d des Führungsdrahts liegt in einem Bereich von etwa 0,15 bis 3 mm, mit Vorteil in einem Bereich von 0,4 bis 2 mm und ganz besonders bevorzugt in einem Bereich von etwa 0,8 bis 1,2 mm. Der Durchmesser d kann über die Länge des Führungsdrahts variieren, ist aber in bevorzugten Ausführungsformen eher konstant. Das Bezugszeichen S gibt schematisch an, dass hier ausgehend von der Auswerteeinheit 10 Steuersignale ausgegeben werden, um ein anderes Gerät wie beispielsweise ein Saug-Spül-System zu regeln oder zu steuern.

### Bezugszeichenliste

- 10: Auswerteeinheit
- 12: Kabel
- 20: Führungsdraht
- 21: distales Ende (des Führungsdrahts)
- 22: proximales Ende (des Führungsdrahts)
- 40: Messeinrichtung
- S: Steuersignal
- d: Durchmesser

## Patentansprüche

1. Überwachungssystem für endoskopische Behandlungen, insbesondere zur Anwendung bei endourologischen Eingriffen,
umfassend eine Auswerteeinheit (10) und einen Führungsdraht (20), welcher zumindest eine Messeinrichtung (40) umfasst,
wobei die Auswerteeinheit (10) eingerichtet ist zum Empfangen von Daten der zumindest einen Messeinrichtung (40) und zur Überwachung der Umgebungsbedingungen in einer Behandlungszone,
**dadurch gekennzeichnet,**
**dass** die Auswerteeinheit (10) eine Vielzahl von Vergleichswerten umfasst, und
**dass** die Auswerteeinheit (10) einen Komparator umfasst, der eingerichtet ist zum Vergleichen der Daten mit der Vielzahl von Vergleichswerten zur Erfassung, ob die Umgebungsbedingungen in einem ungünstigen Bereich liegen, und
**dass** zumindest einer der Vergleichswerte ein Relativwert ist, der sich auf ein Start-Druckniveau bezieht, der in der entsprechenden Behandlungszone vor Beginn der Behandlung herrscht, und
**dass** die Auswerteeinheit (10) eine Eingabeeinheit umfasst, die dazu eingerichtet ist, die Vielzahl der Vergleichswerte anzupassen.

2. Überwachungssystem nach Anspruch 1,
wobei die zumindest eine Messeinrichtung (40) dazu eingerichtet ist, einen Druck oder eine Druckänderung und/oder eine Temperatur oder eine Temperaturänderung zu erfassen.

3. Überwachungssystem nach einem der vorhergehenden Ansprüche, wobei die Auswerteeinheit (10) eine Ausgabeeinrichtung umfasst, die dazu eingerichtet ist, ein Ausgabesignal zu erzeugen, welches erkennen lässt, wenn der Vergleichswert überschritten wird.

4. Überwachungssystem nach Anspruch 4, wobei die Ausgabeeinrichtung dazu eingerichtet ist, unterschiedliche Ausgabesignale zu erzeugen, abhängig davon, welcher Vergleichswert überschritten wird.

5. Überwachungssystem nach einem der vorhergehenden Ansprüche, wobei die Auswerteeinheit (10) dazu eingerichtet ist zu erfassen, wie oft und/oder wie lange und/oder wie weit der Vergleichswert überschritten wird.

6. Überwachungssystem nach einem der vorhergehenden Ansprüche, wobei die Auswerteeinheit dazu eingerichtet ist, ein Steuersignal (S) für ein anderes Gerät, insbesondere für ein Saug-Spül-System, zu erzeugen, wenn einer der Vergleichswerte überschritten wird.

7. Überwachungssystem nach einem der vorhergehenden Ansprüche, wobei die zumindest eine Messeinrichtung (40) dazu eingerichtet ist, durch die Auswerteeinheit (10) kalibriert zu werden, insbesondere indem ein Druck- und/oder Temperaturniveau auf Null gesetzt wird.

8. Überwachungssystem nach einem der vorhergehenden Ansprüche, wobei die zumindest eine Messeinrichtung (40) an einem distalen Ende (21) des Führungsdrahts (20) angeordnet ist.

9. Überwachungssystem nach einem der vorhergehenden Ansprüche, wobei zumindest eine Messeinrichtung (40) ein relativer Drucksensor und zumindest eine weitere Messeinrichtung (40) ein absoluter Drucksensor ist, und
wobei der relative Drucksensor bevorzugt an einem distalen Ende (21) des Führungsdrahts (20) angeordnet ist, während der absolute Drucksensor bevorzugt an einem proximalen Ende (22) des Führungsdrahts (20) angeordnet ist.

10. Überwachungssystem nach einem der vorhergehenden Ansprüche, wobei zumindest eine Messeinrichtung (40) ein Drucksensor und zumindest eine weitere Messeinrichtung (40) ein Temperatursensor ist.

## Claims

1. A monitoring system for endoscopic treatments, in particular for use in en-dourological interventions,
comprising an evaluation unit (10) and a guidewire (20), which comprises at least one measuring device (40),
wherein the evaluation unit (10) is adapted to receiving data of the at least one measuring device (40) and to monitoring the ambient conditions in a treatment zone,
**characterized in**
**that** the evaluation unit (10) comprises a plurality of comparison values, and that the evaluation unit (10) comprises a comparator, which is adapted to comparing the data with the plurality of comparison values to determine whether the ambient conditions lie within an unfavorable range, and
**that** at least one of the comparison values is a relative value, which relates to a starting pressure level which prevails in the corresponding treatment zone prior to the start of the treatment, and
**that** the evaluation unit (10) comprises an input unit which is adapted to adjust the plurality of comparison values.

2. The monitoring system according to claim 1,
wherein the at least one measuring device (40) is adapted to acquire a pressure or pressure change and/or a temperature or temperature change.

3. The monitoring system according to any one of the preceding claims,
wherein the evaluation unit (10) comprises an output unit, which is adapted to generate an output signal that indicates when the comparison value is exceeded.

4. The monitoring system according to claim 4,
wherein the output device is adapted to generate various output signals, depending on which comparison value is exceeded.

5. The monitoring system according to any one of the preceding claims, wherein the evaluation unit (10) is adapted to determine how often and/or how long and/or by how much the comparison value is exceeded.

6. The monitoring system according to any one of the preceding claims, wherein the evaluation unit is adapted to generate a control signal (S) for another device, in particular for a suction-rinsing system, when one the comparison values is exceeded.

7. The monitoring system according to any one of the preceding claims, wherein the at least one measuring device (40) is adapted to be calibrated by the evaluation unit (10), in particular by setting a pressure and/or temperature level to zero.

8. The monitoring system according to any one of the preceding claims, wherein the at least one measuring device (40) is arranged at a distal end (21) of the guidewire (20).

9. The monitoring system according to any one of the preceding claims, wherein at least one measuring device (40) is a relative pressure sensor, and at least one additional measuring device (40) is an absolute pressure sensor, and
wherein the relative pressure sensor is preferably arranged at a distal end (21) of the guidewire (20), while the absolute pressure sensor is preferably arranged at a proximal end (22) of the guidewire (20).

10. The monitoring system according to any one of the preceding claims, wherein at least one measuring device (40) is a pressure sensor, and at least one additional measuring device (40) is a temperature sensor.

## Revendications

1. Système de surveillance destiné à des traitements endoscopiques, en particulier à l'application dans des interventions endo-urologiques,
comprenant une unité d'évaluation (10) et un fil de guidage (20) qui comprend au moins un dispositif de mesure (40),
dans lequel
l'unité d'évaluation (10) est conçue pour recevoir des données dudit au moins un dispositif de mesure (40) et pour surveiller les conditions ambiantes dans une zone de traitement,
**caractérisé en ce que**
l'unité d'évaluation (10) comprend une multitude de valeurs de comparaison, et l'unité d'évaluation (10) comprend un comparateur qui est conçu pour comparer les données avec la multitude de valeurs de comparaison afin de détecter si les conditions ambiantes sont dans une plage défavorable, et
l'une au moins des valeurs de comparaison est une valeur relative qui se réfère à un niveau de pression de départ qui règne dans la zone de traitement correspondante avant le début du traitement, et
l'unité d'évaluation (10) comprend une unité d'entrée conçue pour adapter la multitude de valeurs de comparaison.

2. Système de surveillance selon la revendication 1,
dans lequel ledit au moins un dispositif de mesure (40) est conçu pour détecter une pression ou une variation de pression et/ou une température ou une variation de température.

3. Système de surveillance selon l'une des revendications précédentes,
dans lequel l'unité d'évaluation (10) comprend un dispositif de sortie conçu pour générer un signal de sortie indiquant si la valeur de comparaison est dépassée.

4. Système de surveillance selon la revendication 4,
dans lequel le dispositif de sortie est conçu pour générer différents signaux de sortie en fonction de savoir quelle valeur de comparaison est dépassée.

5. Système de surveillance selon l'une des revendications précédentes,
dans lequel l'unité d'évaluation (10) est conçue pour détecter la fréquence et/ou la durée et/ou l'ampleur du dépassement de la valeur de comparaison.

6. Système de surveillance selon l'une des revendications précédentes,
dans lequel l'unité d'évaluation est conçue pour générer un signal de commande (S) pour un autre dispositif, en particulier pour un système d'aspiration-rinçage, si l'une des valeurs de comparaison est dépassée.

7. Système de surveillance selon l'une des revendications précédentes,
dans lequel ledit au moins un dispositif de mesure (40) est conçu pour être calibré par l'unité d'évaluation (10), en particulier en mettant à zéro un niveau de pression et/ou de température.

8. Système de surveillance selon l'une des revendications précédentes,
dans lequel ledit au moins un dispositif de mesure (40) est disposé à une extrémité distale (21) du fil de guidage (20).

9. Système de surveillance selon l'une des revendications précédentes,
dans lequel au moins un dispositif de mesure (40) est un capteur de pression relative et au moins un autre dispositif de mesure (40) est un capteur de pression absolue, et
dans lequel le capteur de pression relative est de préférence disposé à une extrémité distale (21) du fil de guidage (20), tandis que le capteur de pression absolue est de préférence disposé à une extrémité proximale (22) du fil de guidage (20).

10. Système de surveillance selon l'une des revendications précédentes,
dans lequel au moins un dispositif de mesure (40) est un capteur de pression et au moins un autre dispositif de mesure (40) est un capteur de température.
